(19) Europäisches Patentamt
European Patent Office
Office européen des brevets

(11) Veröffentlichungsnummer: **0 071 958**
**B1**

(12) # EUROPÄISCHE PATENTSCHRIFT

(45) Veröffentlichungstag der Patentschrift: **10.04.85**

(21) Anmeldenummer: **82106996.0**

(22) Anmeldetag: **03.08.82**

(51) Int. Cl.⁴: **C 07 D 239/42,** C 07 D 239/46, C 07 D 239/52, C 07 D 251/16, C 07 D 251/46, A 01 N 47/36, C 07 D 493/08 // (C07D493/08, 307:00, 307:00)

(54) **Heterocyclisch substituierte Sulfonylharnstoffe, Verfahren zu ihrer Herstellung und ihre Verwendung in der Landwirtschaft.**

(30) Priorität: **08.08.81 DE 3131489**

(43) Veröffentlichungstag der Anmeldung: **16.02.83 Patentblatt 83/7**

(45) Bekanntmachung des Hinweises auf die Patenterteilung: **10.04.85 Patentblatt 85/15**

(84) Benannte Vertragsstaaten: **AT BE CH DE FR GB IT LI NL SE**

(56) Entgegenhaltungen:
**EP - A - 0 061 661**
**US - A - 4 127 405**

(73) Patentinhaber: **HOECHST AKTIENGESELLSCHAFT, Postfach 80 03 20, D-6230 Frankfurt am Main 80 (DE).**

(72) Erfinder: **Willms, Lothar, Dr., Grüner Weg 4, D-5463 Unkel (DE)**
Erfinder: **Hüttelmaier, Thomas, Dr., Kuckucksweg 19, D-6233 Kelkheim (Taunus) (DE)**
Erfinder: **Mildenberger, Hilmar, Dr., Fasanenstrasse 24, D-6233 Kelkheim (Taunus) (DE)**
Erfinder: **Bauer, Klaus, Dr., Kolpingstrasse 7, D-6054 Rodgau (DE)**
Erfinder: **Bürstell, Helmut, Dr., Am Hohlacker 65, D-6000 Frankfurt am Main 50 (DE)**
Erfinder: **Bieringer, Hermann, Dr., Eichenweg 26, D-6239 Eppstein/Taunus (DE)**

EP 0 071 958 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bereits bekannt, dass heterocyclisch substituierte Phenylsulfonylharnstoffe, wie z.B. N-(4-Chlor-6-i-propylamino-1,3,5-triazin-2-yl)-N-i-propyl-N'-(4-chlorphenylsulfonyl)harnstoff, herbizide oder pflanzenwuchsregulierende Eigenschaften aufweisen (vgl. NL-PS Nr. 121788, DE-OS Nr. 2715786, EP Nrn. 1485, 1514, 1515, 4163, 7687, 9419, 10560, 23140, 23141, 23422).

Es wurde nun gefunden, dass auch heterocyclisch substituierte Cyclo- bzw. Bicycloalkylsulfonylharnstoffe als Herbizide und Pflanzenwachstumsregulatoren geeignet sind.

Gegenstand der vorliegenden Erfindung sind demnach Verbindungen der Formel I

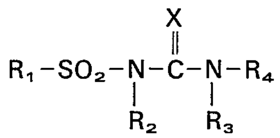

(I)

worin

$R_1$ einen gesättigten cycloaliphatischen Rest mit 3 bis 12 C-Atomen oder einen ein- oder mehrfach ungesättigten cycloaliphatischen Rest mit 5 bis 12 C-Atomen, die ggf. durch bis zu 4 Halogenatome und/oder durch einen oder mehrere $(C_1-C_4)$-Alkyl- oder Halogenalkyl (letztere mit 1 bis 3 Halogenatomen) bzw. durch einen $(C_1-C_4)$-Alkoxycarbonylrest substituiert sein können; einen bicyclischen gesättigten oder 1- oder 2fach ungesättigten aliphatischen Rest mit 7 bis 12 C-Atomen, welcher ggf. bis zu 6 Halogenatome oder eine oder mehrere $(C_1-C_4)$-Alkylreste tragen kann oder in dem eine $-CH_2-$ Brücke durch Sauerstoff ersetzt sein kann,

$R_2$, $R_3$, H oder $(C_1-C_4)$-Alkyl,

X O oder S, und

$R_4$ einen 2 bis 3 Stickstoffatome enthaltenden heterocyclischen Sechsring, der ggf. 1- bis 3fach durch Halogen, $NO_2$, CN, CHO, $(C_1-C_4)$-Alkylamino, $(C_1-C_4)$-Dialkylamino, einen (ggf. durch Halogen, $(C_1-C_3)$-Alkoxy, $(C_1-C_3)$-Alkylthio, $(C_1-C_3)$-Alkylamino, $(C_1-C_3)$-Dialkylamino oder $(C_1-C_4)$-Alkoxycarbonyl substituierten) $(C_1-C_4)$-Alkylrest, einen (ggf. durch Halogen oder $(C_1-C_4)$-Alkoxycarbonyl substituierten) $(C_1-C_4)$-Alkoxy- oder $(C_1-C_4)$-Alkylthiorest oder $(C_1-C_4)$-Alkoxycarbonyl substituiert ist, bedeuten,

sowie, falls $R_2$ Wasserstoff bedeutet, deren physiologisch verträgliche Salze mit Basen.

Halogen bedeutet bevorzugt Fluor, Chlor oder Brom.

Besonders bevorzugt sind Verbindungen in denen $R_1$ einen gesättigten oder einfach ungesättigten, unsubstituierten oder durch Cl oder $CH_3$ ein- oder mehrfach substituierten $(C_5-C_8)$-cycloaliphatischen oder $(C_7-C_8)$-bicyclischen Rest oder einen durch Chlor oder Brom substituierten $(C_6-C_{12})$-Cycloalkylrest bedeuten, wobei Cl vorzugsweise bis zu dreimal, $CH_3$ bis zu 9mal vorhanden sein können.

Als Beispiele für erfindungsgemässe heterocyclisch substituierte Sulfonylharnstoffe der Formel I seien – zusätzlich zu den im experimentellen Teil beschriebenen Verbindungen – die folgenden genannt:

N-[(4-Methoxy-6-methyl-1,3,5-triazin-2-yl)aminocarbonyl]-1,2-dichlorcyclohexylsulfonamid

N-[(2,6-Dimethyl-5-chlorpyrimidin-2-yl)aminocarbonyl]-3-bromcyclohex-1-enylsulfonamid

N-[(4-Methyl-6-methylthio-1,3,5-triazin-2-yl)aminocarbonyl]cyclohexa-1,3-dienylsulfonamid

N-[(4-Methyl-6-dimethylamino-1,3,5-triazin-2-yl)aminocarbonyl]-2-chlorcyclopenthylsulfonamid

N-[(5,6-Dimethyl-1,2,4-triazin-3-yl)aminothiocarbonyl]cyclopent-1-enylsulfonamid

N-[(4,6-Dimethoxy-5-chlorpyrimidin-2-yl)-methylaminocarbonyl]cyclopent-1-enylsulfonamid

N-[(4,5-Dimethyl-6-methoxypyrimidin-2-yl)aminocarbonyl]cyclohept-1-enylsulfonamid

N-[(4-Methyl-5-nitro-6-chlorpyrimidin-2-yl)aminocarbonyl]-2-chlorcyclooctylsulfonamid

N-[(4-Methoxycarbonyl-6-methyl-1,3,5-triazin-2-yl)aminocarbonyl]cyclooct-1-enylsulfonamid

N-[(4-Chlor-6-isopropylamino-1,3,5-triazin-2-yl)aminocarbonyl]cyclooct-1-enylsulfonamid

N-[(4-Trifluormethyl-6-methylpyrimidin-2-yl)aminocarbonyl]cyclohex-3-enylsulfonamid

N-[(4,6-Dimethylmercapto-1,3,5-triazin-2-yl)aminocarbonyl]cyclohexylsulfonamid

N-[(4-Methylpyrimidin-2-yl)aminocarbonyl]-3,4-dichlorcyclohexylsulfonamid

N-[(4-Methoxy-5-n-butyl-6-methylpyrimidin-2-yl)aminocarbonyl]-1,2-dibromcyclohexylsulfonamid

N-[(4-Methoxycarbonylmethoxy)-6-methylpyrimidin-2-yl)aminocarbonyl]cyclohex-1-enylsulfonamid, Natrium-Salz

N-[(4-Methoxy-6-methyl-1,3,5-triazin-2-yl)-methylaminocarbonyl]cyclohex-1-enylsulfonamid

N-[(4-Methoxy-6-methylpyrimidin-2-yl)-aminocarbonyl]cyclopentylsulfonamid

N-[(4-Ethyl-6-methoxy-1,3,5-triazin-2-yl)-aminocarbonyl]cyclopentylsulfonamid

N-[(4-Methoxy-6-methylpyrimidin-2-yl)aminocarbonyl]-1,2-dichlorcyclopentylsulfonamid

N-[(4-Methoxy-6-methylpyrimidin-2-yl)aminocarbonyl]-2-chlorcyclopentylsulfonamid

N-[(4,6-Dimethoxy-1,3,5-triazin-2-yl)aminocarbonyl]-2-chlorcyclododecylsulfonamid

N-[(4-Methoxy-6-methylpyrimidin-2-yl)aminocarbonyl]-3-chlorbicyclo-[2.2.1]-hept-2-ylsulfonamid

N-[(4-Methoxy-6-methyl-1,3,5-triazin-2-yl)aminocarbonyl]bicyclo-[2.2.1]-hept-5-en-2-ylsulfonamid

N-[(4,6-Dimethylpyrimidin-2-yl)aminocarbonyl]bicyclo-[2.2.2]-oct-2-ylsulfonamid

N-[(4-Methoxy-6-methyl-1,3,5-triazin-2-yl)aminocarbonyl]-2,3,3-trimethylbicyclo-[2.2.1]-hept-2-ylsulfonamid

N-[(4-Methoxy-6-methylpyrimidin-2-yl)aminocarbonyl]bicyclo-[2.2.1]-hept-2-ylsulfonamid

N-[(5,6-Dimethyl-1,2,4-triazin-3-yl)aminocarbonyl]-(4,5-Dichlorbicyclo-[2.2.1]-heptyl)sulfonamid

N-[(4,6-Dimethylpyrimidin-2-yl)aminocarbonyl]-2,2,5,5-tetramethylcyclohex-3-enylsulfonamid

N-[(4-Methoxymethyl-6-methyltriazin-2-yl)aminocarbonyl]-2-chlorcyclohexenylsulfonamid

N-[(4-Methoxy-6-methylpyrimidin-2-yl)aminocarbonyl]-2-chlor-3-methylcyclohex-4-enylsulfonamid

N-[(4,6-Dimethyl-1,3,5-triazin-2-yl)aminocarbonyl]-2,4,5-trichlor-3-methylcyclohexylsulfonamid

N-[(4-Methoxy-6-methylpyrimidin-2-ylaminocarbonyl]-2-chlorcyclooctylsulfonamid

N-[(4,6-Dimethyl-1,3,5-triazin-2-yl)aminocarbonyl]cyclopropylsulfonamid

N-[(4-Methoxy-6-methyl-1,3,5-triazin-2-yl)aminocarbonyl]-2-methyl-5-isopropylcyclohexylsulfonamid

N-[(4,6-Dimethyl-1,3-pyrimidin-2-yl)aminocarbonyl]bicyclo-[3.2.2]-non-2-ylsulfonamid

Die neuen Verbindungen der allgemeinen Formel I lassen sich aus an sich bekannten bzw. nach bekannten Verfahren hergestellten Ausgangsmaterialien synthetisieren. Die Herstellungsverfahren sind dadurch gekennzeichnet, dass man

a) Verbindungen der Formel

$$R_1-SO_2-N=C=O \qquad (II)$$

oder

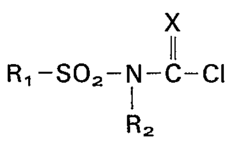

(III)

mit Verbindungen der Formel

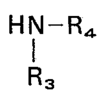

(IV)

oder

b) Verbindungen der Formel

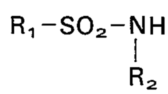

(V)

mit Verbindungen der Formel

$$S=C=N-R_4 \qquad (VI)$$

oder

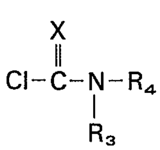

(VII)

worin $R_3$ $(C_1-C_4)$-Alkyl bedeutet,

oder

c) Verbindungen der Formel

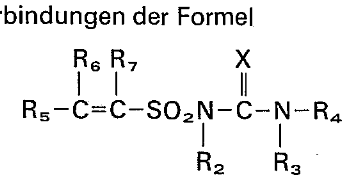

(VIII)

worin $R_5$, $R_6$ und $R_7$ Wasserstoff, Halogen oder $(C_1-C_4)$-Alkyl bedeuten

mit Verbindungen der Formel

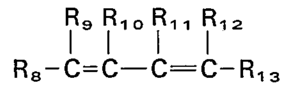

(IX)

worin $R_8$ bis $R_{12}$ für Wasserstoff, Halogen oder $CH_3$ stehen und einer der Reste $R_9$ bis $R_{12}$ auch $(C_2-C_4)$-Alkyl oder $(C_1-C_4)$-Alkoxycarbonyl sein kann oder $R_8$ und $R_{13}$ zusammen eine $(C_1-C_6)$-Alkylengruppe, in der eine $-CH_2$-Gruppe auch durch Sauerstoff ersetzt sein kann, darstellen, umsetzt und gewünschtenfalls die erhaltenen Verbindungen der Formel I durch Abspaltung von Halogenwasserstoff, Anlagerung von Halogen oder Wasserstoff an vorhandene Mehrfachbindungen, Alkylierung in $R_2$-Position oder Salzbildung in andere Verbindungen der Formel I überführt.

Zu a) Die Umsetzung der Verbindungen II bzw. III und IV erfolgt vorzugsweise in inerten aprotischen Lösungsmitteln, wie z. B. Acetonitril, Dichlormethan, Toluol, Tetrahydrofuran oder Dioxan, bei Temperaturen zwischen 0° C und der Siedetemperatur des Lösungsmittels. Bei Anwendung von Ausgangsstoffen der Formel III arbeitet man in Gegenwart eines Säureakzeptors, wie z. B. Kaliumcarbonat, Pyridin oder Triethylamin.

Zu b) Bei der Umsetzung der Verbindungen V mit VI bzw. VII arbeitet man gleichfalls in den oben genannten inerten Lösungsmitteln unter Zusatz basischer Verbindungen, wie z. B. Kaliumcarbonat, Pyridin oder Triethylamin bei Temperaturen zwischen 0° C und der Siedetemperatur des Lösungsmittels.

Zu c) Die Umsetzung der Verbindungen VIII und IX erfolgt vorzugsweise in inerten Lösungsmitteln, wie z. B. Toluol, Xylol, Dioxan oder Dichlormethan, bei Temperaturen zwischen Raumtemperatur und der Siedetemperatur des Lösungsmittels. Ggf. kann die Reaktion in Anwesenheit von Katalysatoren wie z. B. Aluminiumtrichlorid oder im Autoklaven unter erhöhtem Druck durchgeführt werden.

Die nachträgliche Abspaltung von Halogenwasserstoff (HCl, HBr) aus halogenhaltigen Resten $R_1$ erfolgt in bekannter Weise, z. B. mit Alkalialkoholat, alkoholischer Natron- oder Kalilauge, Triethylamin oder anderen säureabspaltenden Mitteln, ggf. in Gegenwart eines weiteren inerten Lösungs- oder Verdünnungsmittels (z. B. Toluol) bei Temperaturen zwischen Raum- und Siedetemperatur.

An vorhandene oder nachträglich gebildete Mehrfachbindungen in $R_1$-Position kann man Halogen ($Cl_2$, $Br_2$), Halogenwasserstoff oder Wasserstoff bei Normaldruck oder unter erhöhtem Druck, ggf. in Anwesenheit eines Katalysators, z.B. Pd/Kohle oder Raney-Nickel in gleichfalls bekannter Weise anlagern und so ggf. zu neuen Verbindungen der Formel I gelangen. Die Bromierung bzw. Chlorierung wird in inerten organischen Lösungsmitteln wie z. B. Dichlormethan oder Chloroform unter Belichtung – beispielsweise mit ultraviolettem Licht – oder in Gegenwart von radikalisch zerfallenden Verbindungen – z. B. Azodiisobutyronitril – bei Temperaturen zwischen 0° C und der Siedetemperatur des Lösungsmittels durchgeführt. Die Anlagerung von Halogenwasserstoff gelingt in Gegenwart inerter Lösungsmittel (z. B. Toluol) mittels gasförmigen HCl oder HBr bei niedrigen Temperaturen, ggf. in Gegenwart eines Peroxidkatalysators.

Zur nachträglichen Alkylierung in $R_2$-Stellung arbeitet man vorzugsweise in inerten Lösungsmitteln wie z. B. Dioxan oder Dimethylformamid unter Zusatz einer anorganischen Base, z. B. Natriumhydrid oder Kaliumcarbonat, bei Temperaturen von 20° C bis zur Siedetemperatur des Lösungsmittels. Als Alkylierungsmittel werden z. B. Dimethylsulfat, Methyljodid oder Ethylbromid eingesetzt. Verbindungen der Formel I, in denen $R_2$ Wasserstoff bedeutet, können Salze bilden, bei denen H durch ein für die Landwirtschaft geeignetes Kation ersetzt ist. Diese Salze sind im allgemeinen Metall-, Ammonium- oder organische Aminsalze und werden vorzugsweise in inerten Lösungsmitteln wie z. B. Wasser, Methanol oder Aceton bei Temperaturen von 20 bis 100° C hergestellt. Geeignete Basen zur Herstellung der erfindungsgemässen Salze sind z. B. Kaliumcarbonat, Ammoniak oder Ethanolamin.

Die Ausgangsstoffe der Formel IV sind bekannt oder lassen sich nach im Prinzip bekannten Verfahren herstellen, z. B. durch Zyklisierung entsprechender Guanidinderivate mit entsprechend substituierten 1,3-Diketonen (vgl. z. B. „The Chemistry of Heterocyclic Compounds", vol. XVI (1962) und supplement I (1970) oder durch Derivatisierung von Cyanurchlorid (vgl. z. B. „The Chemistry of Heterocyclic Compounds", L. Rapoport: „s-Triazines and Derivatives" [1959]).

Die Sulfonylisocyanate der Formel II sind ebenfalls zum grossen Teil bekannt oder lassen sich nach im Prinzip bekannten Verfahren in einfacher Weise herstellen (vgl. DE-AS Nrn. 1211165, 1230016, 1297601).

Die Sulfonylcarbamoyl- bzw. -thiocarbamoylchloride der Formel III lassen sich nach üblichen Methoden aus den Alkalisalzen der entsprechenden Sulfonamide der Formel V, welche aus der Literatur bekannt sind, durch Umsetzung mit Phosgen oder Thiophosgen darstellen.

Die für die Umsetzungen nach b benötigten Isothiocyanate der Formel VI sind bekannt oder nach bekannten Verfahren zugänglich (vgl. „Tetrahedron", *29*, 691 (1973); Japan Kokai Sho-51-143686).

Gleiches gilt für die heterocyclischen Carbamoylchloride und Thiocarbamoylchloride der Formel VII (vgl. z. B. DE-AS Nrn. 1149718 und 2238870).

Die α,β-ungesättigten Sulfonylharnstoffe der Formel VIII sind in der Patentanmeldung P Nr. 3111451.2 beschrieben.

Die erfindungsgemässen heterocyklischen Sulfonylharnstoffderivate weisen eine ausgezeichnete herbizide Wirkung und in wichtigen Grosskulturen eine sehr gute Selektivität auf. Sie eignen sich daher zur selektiven Bekämpfung zweikeimblättriger und grasartiger annueller und perennierender Unkräuter, insbesondere in landwirtschaftlich bedeutenden Kulturen wie z. B. Weizen, Gerste, Roggen, Reis, Mais, Zuckerrübe und Soja. Dabei ist es gleichgültig, ob die Substanzen in Vorsaat-, Vorauflauf- oder Nachauflaufspritzung ausgebracht werden.

Werden die erfindungsgemässen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schliesslich nach 3 bis 5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Darüberhinaus weisen die erfindungsgemässen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Des weiteren eignen sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine grosse Rolle, da das Lagern hierdurch verringert oder völlig verhindert werden kann. Besonders hervorzuheben ist die wachstumsregulatorische Wirksamkeit der Verbindungen als Wuchshemmer in Getreibe, Mais, Soja, Baumwolle, Rasen sowie ihre Fähigkeiten, den Gehalt an erwünschten Inhaltsstoffen wie Kohlehydraten und Protein bei Nutzpflanzen zu erhöhen. Schliesslich zeigen die Verbindungen eine sehr gute Verbesserung der Fruchtabszission insbesondere bei Zitrusfrüchten oder Reduktion der Haltekraft.

Gegenstand der Erfindung sind daher auch herbizide bzw. wachstumsregulierende Mittel, die gekennzeichnet sind durch einen Gehalt an einer Verbindung der Formel I in Kombination mit üblichen Formulierungshilfsmitteln und Inertstoffen sowie deren Verwendung im landwirtschaftlichen Bereich.

Die erfindungsgemässen Mittel enthalten die Wirkstoffe der Formel I im allgemeinen zu 2 bis 95 Gew.-%. Sie können als Spritzpulver, emulgierbare Konzentrate, versprühbare Lösungen, Stäubemittel oder Granulate in den üblichen Zubereitungen angewendet werden.

Die Spritzpulver sind in Wasser gleichmässig dispergierbare Präparate, die neben dem Wirkstoff ausser einem Verdünnungsmittel oder Inertstoff

noch Netzmittel, z. B. polyoxethylierte Alkylphenole, polyoxethylierte Fettalkohole, Alkyl- oder Alkylphenylsulfonate, und Dispergiermittel, z. B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

Alkylarylsulfonsaure Kalziumsalze wie Cadodecylbenzosulfonat oder nichtionische Emulgatoren wie fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitfettsäureester oder Polyoxethylensorbitester.

Stäubemittel erhält man durch Vermahlen des Wirkstoffes mit fein verteilten, festen Stoffen, z. B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise – gewünschtenfalls in Mischung mit Düngemitteln – hergestellt werden.

Bei herbiziden Mitteln können die Konzentrationen der Wirkstoffe in den handelsüblichen Formulierungen verschieden sein.

In Spritzpulvern variiert die Wirkstoffkonzentration z. B. zwischen etwa 10 und 80%, der Rest besteht aus den oben angegebenen Formulierungszusätzen. Bei emulgierbaren Konzentraten kann die Wirkstoffkonzentration gleichfalls etwa 10 bis 80% betragen. Staubförmige Formulierungen enthalten etwa 2 bis 20%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate ggf. in üblicher Weise verdünnt, z. B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äusseren Bedingungen wie Temperatur, Feuchtigkeit, u. a. variiert die erforderliche Aufwandmenge. Sie beträgt im allgemeinen zwischen 0,01 und 10 kg/ha, vorzugsweise etwa 0,1 bis 5,0 kg/ha Wirkstoff.

Für einige Anwendungsgebiete kann es zweckmässig sein, die neuen Herbizide mit einem oder mehreren Herbiziden gemeinsam anzuwenden, z. B. als Tankmischung oder in Form einer Fertigformulierung, um weitere vorteilhafte Wirkungen zu erzielen.

Die erfindungsgemässen Wirkstoffe können mit anderen Herbiziden, Insektiziden und Fungiziden kombiniert werden.

Zur Anwendung als Wachstumsregulatoren eignen sich Konzentrationen zwischen 0,01 und 1,25 kg/ha. Bevorzugt verwendet man wässerige Dispersionen von Spritzpulvern oder Verdünnungen von emulgierbaren Konzentraten. Die Anwendung erfolgt im Nachauflauf. Bevorzugte Kulturen sind Mais und Tabak.

*Herstellungsbeispiel*

*Beispiel 1:*

*N-[(4-Methoxy-6-methylpyrimidin-2-yl)aminocarbonyl]-2-chlorcyclohexylsulfonamid*

41,7 g (0,3 mol) 2-Amino-4-methoxy-6-methylpyrimidin werden in 500 ml Dichlormethan suspendiert und bei 0° C tropfenweise mit einer Lösung von 71,5 g (0,32 mol) 2-Chlorcyclohexylsulfonylisocyanat in 200 ml Dichlormethan versetzt.

Man rührt 18 h bei Raumtemperatur nach, kühlt das Reaktionsgemisch auf 0° C und versetzt mit n-Hexan. Das ausgefallene Reaktionsprodukt wird abgesaugt und mit n-Hexan gewaschen.

Man erhält 84,9 g (78% d.Th.) an N-[(4-Methoxy-6-methylpyrimidin-2-yl)aminocarbonyl]-2-chlorcyclohexylsulfonamid vom Schmelzpunkt 145 bis 148° C.

*Beispiel 2:*

*N-[(4-Methoxy-6-methylpyrimidin-2-yl)aminocarbonyl]cyclohex-1-enylsulfonamid*

36,2 g (0,1 mol) N-[(4-Methoxy-6-methylpyrimidin-2-yl)aminocarbonyl]-2-chlorcyclohex-1-ylsulfonamid (s. Beispiel 1) werden in 300 ml Methanol suspendiert und bei Raumtemperatur mit 8 g (0,2 mol) Natriumhydroxid – gelöst in 40 ml Wasser – versetzt. Anschliessend wird das Reaktionsgemisch 8 h unter Rückfluss gerührt, im Vakuum eingeengt und in 250 ml Wasser aufgenommen. Nach Filtrieren und Ansäuern mit 2N-HCl auf pH 5 wird mit Essigester extrahiert, anschliessend werden ode Extrakte über Natriumsulfat getrocknet und eingeengt. Nach Zugabe von n-Hexan erhält man 17,2 g (52,7% d.Th.) an N-[(4-Methoxy-6-methylpyrimidin-2-yl)aminocarbonyl]-cyclohex-1-enylsulfonamid vom Schmelzpunkt 163 bis 166° C.

*Beispiel 3:*

*N-[(4,6-Dimethylpyrimidin-2-yl)aminocarbonyl]-7-oxabicyclo-[2.2.1]-hept-2-en-6-ylsulfonamid*

30,7 g (0,25 mol) 2-Amino-4,6-dimethylpyrimidin werden in 350 ml Dichlormethan gelöst und

bei 0° C unter Rühren mit einer Lösung von 52 g (0,26 mol) 7-Oxabicyclo-[2.2.1]-hept-2-en-6-ylsulfonylisocyanat (Diels-Alder-Addukt von Vinylsulfonylisocyanat und Furan) in 100 ml Dichlormethan versetzt. Man rührt 12 h bei Raumtemperatur nach, kühlt auf 0° C versetzt mit n-Hexan. Das angefallene Reaktionsprodukt wird abgesaugt, mit n-Hexan gewaschen und getrocknet. Man erhält 73,6 g (90,8% d.Th.) an N-[(4,6-Dimethylpyrimidin-2-yl)aminocarbonyl]-7-oxabicyclo-[2.2.1]-hept-2-en-6-yl-sulfonamid vom Schmelzpunkt 125 bis 145° C.

*Beispiel 4:*

*N-[(4-Methylthio-6-methyl-1,3,5-triazin-2-yl)aminocarbonyl]-3 (bzw. 4)-methylcyclohex-3-enylsulfonamid*

7,81 g (0,05 mol) 2-Amino-4-methylthio-6-methyl-1,3,5-triazin werden in 150 ml Dichlormethan suspendiert und bei 0° C mit einer Lösung von 11,05 g (0,055 mol) 3 (bzw. 4)-Methylcyclohex-3-en-1-ylsulfonylisocyanat (Isomerengemisch; Diels-Alder-Addukt von Isopren an Vinylsulfonylisocyanat) in 50 ml Dichlormethan versetzt. Man rührt 18 h bei Raumtemperatur nach und arbeitet analog Beispiel 1 auf. Man erhält 13,2 g (74% d.Th.) an N-[(4-Methylthio-6-methyl-1,3,5-triazin-2-yl)aminocarbonyl]-3 (bzw. 4)-methylcyclohex-3-enylsulfonamid vom Schmelzpunkt 156 bis 160° C.

*Beispiel 5:*

*N-[(4,6-Dimethylpyrimidin-2-yl)aminocarbonyl]-2-chlorcycloheptylsulfonamid*

14,3 g (0,06 mol) 2-Chlorcycloheptylsulfonylisocyanat werden in 100 ml Dichlormethan vorgelegt und bei 0° C portionsweise mit 7,4 g (0,06 mol) 2-Amino-4,6-dimethylpyrimidin versetzt. Man rührt zunächst 2 h bei 0° C und dann 18 h bei Raumtemperatur nach. Danach extrahiert man die organische Phase mit 3 × 40 ml 2N-$H_2SO_4$, wäscht neutral und trocknet die organische Schicht über $Na_2SO_4$. Dann versetzt man mit 50 ml n-Hexan und zieht das Lösungsmittel ab. Man erhält 13,3 g (61% d.Th.) an N-[(4,6-Dimethylpyrimidin-2-yl)aminocarbonyl]-2-chlorcycloheptylsulfonamid (viskoses Öl).

*Beispiel 6:*

*N-[(4,6-Dimethylpyrimidin-2-yl)aminocarbonyl]-2-chlorcyclododecylsulfonamid*

15,4 g (0,05 mol) 2-Chlordodecylsulfonylisocyanat werden in 100 ml Dichlormethan vorgelegt und bei 0° C portionsweise mit 6,2 g (0,05 mol) 2-Amino-4,6-dimethylpyrimidin versetzt. Man rührt zunächst 2 h bei 0° C und dann 18 h bei Raumtemperatur nach und arbeitet analog Beispiel 5 auf. Man erhält 15,2 g (71% d.Th.) an N-[(4,6-Dimethylpyrimidin-2-yl)aminocarbonyl]-2-chlorcyclododecylsulfonamid (hellgelbe Festsubstanz, Smp. 84° C).

*Beispiel 7:*

*N-[(4,6-Dimethylpyrimidin-2-yl)aminocarbonyl]-3-chlorbicyclo-[2.2.1]-hept-2-ylsulfonamid*

14,1 g (0,06 mol) 3-Chlorbicyclo-[2.2.1]-hept-2-ylsulfonylisocyanat werden in 100 ml Dichlormethan vorgelegt und bei 0° C portionsweise mit 7,4 g (0,06 mol) 2-Amino-4,6-dimethylpyrimidin versetzt. Man rührt zunächst 2 h bei 0° C und dann 18 h bei Raumtemperatur nach und arbeitet analog Beispiel 5 auf. Man erhält 14,1 g (66% d.Th.) an N-[(4,6-Dimethylpyrimidin-2-yl)aminocarbonyl]-3-chlorbicyclo-[2.2.1]-hept-2-ylsulfonamid (viskoses Öl).

In analoger Weise werden erhalten.

*Tabelle 1*

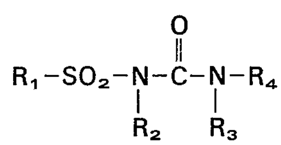 (I)

| Beispiel Nr. | $R_1$ | $R_2$ | $R_3$ | $R_4$ | Smp. (° C) |
|---|---|---|---|---|---|
| 8 | 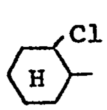 | H | H | 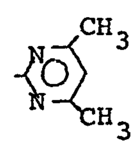 | 155-163 |
| 9 | » | H | H | 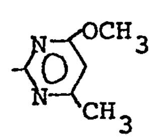 | 118-123 |
| 10 | » | H | H | 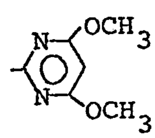 | 159-162 |

*Tabelle 1 (Fortsetzung)*

| Beispiel Nr. | $R_1$ | $R_2$ | $R_3$ | $R_4$ | Smp. (° C) |
|---|---|---|---|---|---|
| 11 | 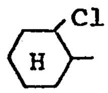 | H | H | 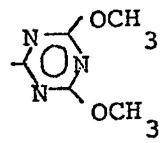 | 143-144 |
| 12 | 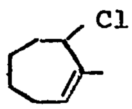 | H | H | 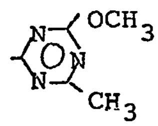 | glasartig |
| 13 | 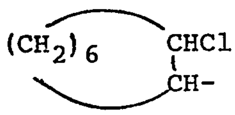 | H | H | 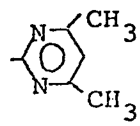 | 48-52 (Zers.) |
| 14 | » | H | H | 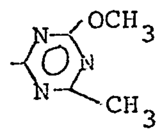 | zähes Öl |
| 15 | 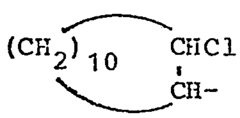 | H | H | 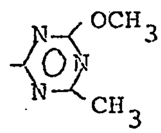 | 103 |
| 16 | 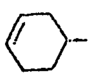 | H | H | 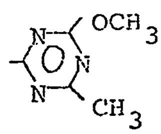 | 142-144 |
| 17 | 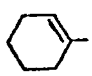 | H | H | 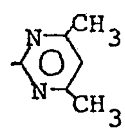 | 165-169 |
| 18 | » | H | H | 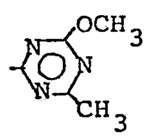 | 150 |
| 19 | » | H | H | 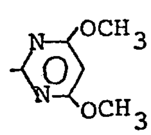 | 159-167 |
| 20 | 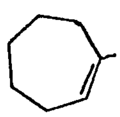 | H | H | 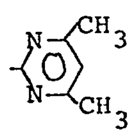 | zähes Öl |
| 21 | » | H | H | 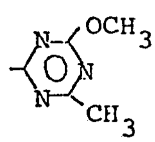 | zähes Öl |
| 22 | 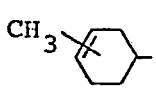 | H | H | 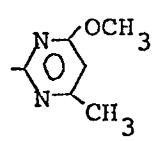 | 195 |

*Tabelle 1 (Fortsetzung)*

| Beispiel Nr. | $R_1$ | $R_2$ | $R_3$ | $R_4$ | Smp. (° C) |
|---|---|---|---|---|---|
| 23 | 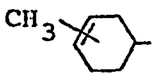 | H | H | 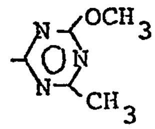 | 143 |
| 24 | » | H | H | 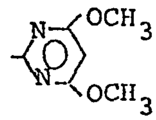 | 173-176 |
| 25 | » | H | H | 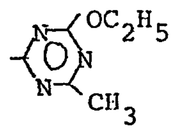 | 89-92 |
| 26 | » | H | H | 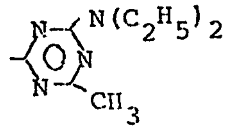 | 161-164 |
| 27 | 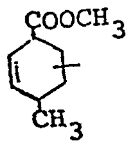 | H | H | 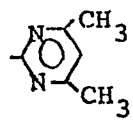 | 54-57 |
| 28 | » | H | H | 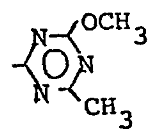 | 88-91 |
| 29 | 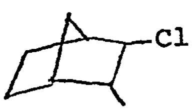 | H | H | 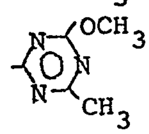 | zähes Öl |
| 30 | 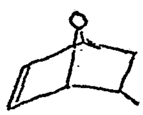 | H | H | 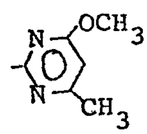 | 146-150 |
| 31 | » | H | H | 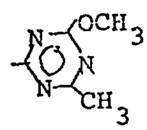 | 135-145 |
| 32 | 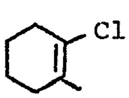 | H | H | 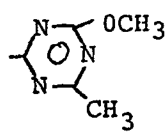 | Harz |
| 33 | 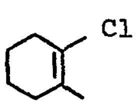 | H | H | 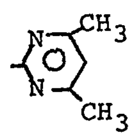 | 152-154 |
| 34 | 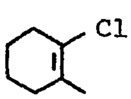 | H | H | 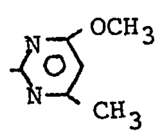 | |

*Tabelle 1 (Fortsetzung)*

| Beispiel Nr. | $R_1$ | $R_2$ | $R_3$ | $R_4$ | Smp. (°C) |
|---|---|---|---|---|---|
| 35 | 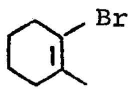 | H | H | 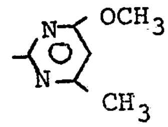 | |
| 36 | 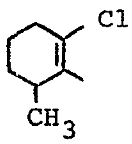 | H | H | 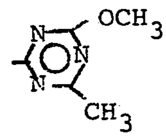 | |
| 37 | 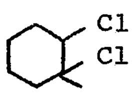 | H | H | 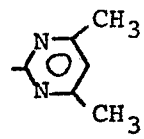 | 78-80 |
| 38 | 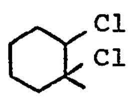 | H | $CH_3$ | 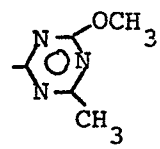 | |
| 39 | 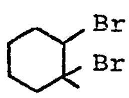 | H | H | 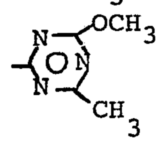 | |
| 40 | 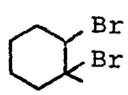 | H | H | 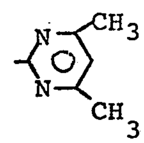 | 137-138 |

*Formulierungsbeispiele*

*Beispiel A:*

Ein emulgierbares Konzentrat wird erhalten aus
15 Gew.-Teilen Wirkstoff
75 Gew.-Teilen Cyclohexan als Lösungsmittel, und
10 Gew.-Teilen oxethyliertes Nonylphenol (10 AeO) als Emulgator.

*Beispiel B:*

Ein in Wasser leicht dispergierbares benetzbares Pulver wird erhalten, indem man
25 Gew.-Teile Wirkstoff
64 Gew.-Teile kaolinhaltiges Quarz als Inertstoff
10 Gew.-Teile ligninsulfonsaures Kalium, und
1 Gew.-Teil oleylmethyltaurinsaures Natrium als Netz- und Dispergiermittel
mischt und in einer Stiftmühle mahlt.

*Beispiel C:*

Ein Stäubemittel wird erhalten, indem man
10 Gew.-Teile Wirkstoff, und
90 Teile Talkum als Inertstoff
mischt und in einer Schlagmühle zerkleinert.

*Beispiel D:*

Ein Granulat besteht z. B. aus etwa
2 bis 15 Gew.-Teile Wirkstoff
98 bis 85 Gew.-Teile inerte Granulatmaterialien, wie z. B. Attapulgit, Bimsstein und Quarzsand.

*Biologische Beispiele*

a) *Herbizide Wirkung*

Die Schädigung der Unkrautpflanzen bzw. die Kulturpflanzenverträglichkeit wurde in einem Schlüssel von 0 bis 5 bonitiert.

Dabei bedeutet
0 = ohne Wirkung (Schaden)
1 = 0-20% Wirkung
2 = 20-40% Wirkung
3 = 40-60% Wirkung
4 = 60-80% Wirkung
5 = 80-100% Wirkung

Die Abkürzungen bedeuten
LOM = Raygras
STM = Sternmiere
SIA = Ackersenf
AS = Aktivsubstanz

1. *Vorlaufverfahren*

Samen bzw. Rhizomstücke mono- und dikotyler Unkräuter wurden in Lehmerde ausgelegt und mit Erde abgedeckt. Die als benetzbare Pulver formulierten erfindungsgemässen Verbindungen wurden in Form wässeriger Suspensionen oder Emulsionen auf die Erdoberfläche appliziert. Die Wasseraufwandmenge pro Topf entsprach dabei umgerechnet 600 bis 800 l/ha. Nach der Behandlung wurden die Versuchstöpfe im Gewächshaus aufgestellt und die Versuchspflanzen unter guten Wachstumsbedingungen (Temperatur: ca. 23° C; rel. Luftfeuchte 60 bis 80%) kultiviert. Nach ca. 3 Wochen wurde die Pflanzenschädigung optisch bonitiert. Als Vergleich dienten dabei unbehandelte Kontrollen.

In Tabelle 2 sind die Vorlaufergebnisse zusammengestellt. Es wird deutlich, dass die erfindungsgemässen Verbindungen eine gute herbizide Wirkung sowohl gegen monokotyle als auch gegen dikotyle Schadpflanzen aufweisen, wenn die Wirkstoffe im Vorlaufverfahren appliziert werden.

*Tabelle 2:*

*Vorlaufwirkung der erfindungsgemässen Verbindungen gegen mono- und dikotyle Unkräuter*

| Beispiele Nr. | Dosis (kg AS/ha) | LOM | STM |
|---|---|---|---|
| 1 | 2,5 | 3 | 4 |
| 9 | 2,5 | 2 | 3 |
| 10 | 2,5 | 3 | 2 |
| 16 | 2,5 | 5 | 5 |
| 19 | 2,5 | 5 | 5 |
| 3 | 2,5 | 4 | 4 |
| 30 | 2,5 | 5 | 5 |
| 31 | 2,5 | 5 | 4 |
| 28 | 2,5 | 2 | 3 |
| 32 | 2,5 | 5 | 5 |
| 33 | 2,5 | 5 | 5 |
| 37 | 2,5 | 5 | 5 |

2. *Nachlaufverfahren*

Samen von mono- und dikotylen Unkräutern wurden in Töpfen ausgesät und im Gewächshaus unter guten Wachstumsbedingungen angezogen. 3 Wochen nach der Aussaat wurden die Versuchspflanzen im 3-Blatt-Stadium behandelt. Die als Spritzpulver bzw. als Emulsionskonzentrate formulierten erfindungsgemässen Präparate wurden in verschiedenen Dosierungen auf die grünen Pflanzenteile gesprüht und nach ca. 3 Wochen Standzeit im Gewächshaus unter optimalen Wachstumsbedingungen (Temperatur: ca. 23° C; rel. Luftfeuchte 60 bis 80%) die Wirkung der Präparate optisch im Vergleich zu unbehandelten Kontrollen bonitiert.

Die erfindungsgemässen Mittel wiesen eine gute herbizide Wirksamkeit gegen ein breites Spektrum wirtschaftlich wichtiger annueller und perennierender Unkräuter und Ungräser auf (Tabelle 3).

*Tabelle 3:*

*Herbizide Wirkung der erfindungsgemässen Verbindungen gegen mono- und dikotyle Unkräuter im Nachauflauf*

| Präparat | Dosis (kg AS/ha) | LOM | SIA |
|---|---|---|---|
| 9 | 2,5 | 1 | 5 |
| 10 | 2,5 | 3 | 4 |
| 19 | 2,5 | 5 | 5 |
| 3 | 2,5 | 3 | 5 |
| 30 | 2,5 | 4 | 5 |
| 31 | 2,5 | 5 | 5 |
| 27 | 2,5 | 0 | 4 |
| 32 | 2,5 | 5 | 5 |
| 33 | 2,5 | 5 | 5 |
| 37 | 2,5 | 5 | 5 |

b) *Pflanzenwuchsregulierende Wirkung*

3. *Wuchshemmung an Getreide*

In Schalenversuchen im Gewächshaus wurden junge Getreidepflanzen (Weizen, Gerste und Roggen) im 3-Blatt-Stadium mit den in Tabelle 1 genannten Verbindungen in den angegebenen Wirkstoffkonzentrationen (kg/ha) tropfnass gespritzt. Als Vergleichsverbindung wurde 2-Chlorethyltrimethylammoniumchlorid eingesetzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde ausserdem die phytotoxische Wirkung der Verbindungen beobachtet. Die Ergebnisse sind in der Tabelle 4 zusammengefasst. Bei der Angabe der Wuchshemmung bedeuten 100% den Stillstand des Wachstums und 0% ein Wachstum entsprechend dem der unbehandelten Kontrollpflanzen.

*Tabelle 4:*

*Wuchshemmung bei Getreide*

| Verbindung gem. Beispiel | Anwendungs-konz. (kg/ha) | Wuchshemmung (%) | | | Phytotox. Wirkung |
|---|---|---|---|---|---|
| | | Weizen | Gerste | Roggen | |
| 3 | 0,62<br>0,31 | 22<br>11 | 24<br>21 | 22<br>18 | keine Schäden |

*Tabelle 4:* ***(Fortsetzung)***

*Wuchshemmung bei Getreide*

| Verbindung gem. Beispiel | Anwendungs-konz. (kg/ha) | Wuchshemmung (%) | | | Phytotox. Wirkung |
|---|---|---|---|---|---|
| | | Weizen | Gerste | Roggen | |
| 28 | 1,25 | 19 | 18 | 15 | keine Schäden |
| 19 | 1,25 | 15 | 10 | 11 | keine Schäden |
| Vergleich: (2-Chlorethyl)tri-methylammoniumchlorid | 2,50<br>1,25 | 27<br>23 | 8<br>0 | 10<br>0 | keine Schäden |

4. *Wuchshemmung an Buschbohnen*

10 bis 15 cm Buschbohnen wurden mit den Wirkstoffzubereitungen tropfnass bespritzt. Nach 2 Wochen wird der Zuwachs gemessen und die Wuchshemmung in % des Zuwachses der Kontrollpflanzen berechnet. Die Ergebnisse sind in Tabelle 5 zusammengefasst.

*Tabelle 5:*

*Wuchshemmung an Buschbohnen*

| Verbindung gem. Beispiel | Anwendungs-konz. (kg/ha) | Wuchs-hemmung (%) | Phytotox. Wirkung |
|---|---|---|---|
| 3 | 0,62<br>0,31 | 40<br>20 | keine Schäden |
| Vergleich: 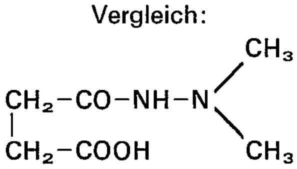 | 2,50 | 34 | keine Schäden |

5. *Wuchshemmung an Rasen*

Eine Rasenmischung, bestehend aus 5 repräsentativen Arten, wird nach dreimaligem Rückschnitt mit einer Wirkstoffzubereitung tropfnass gespritzt. Nach 3 bis 4 Wochen wird der Zuwachs gemessen und die Wuchshemmung in % des Zuwachses der Kontrollpflanzen berechnet. Es bedeuten 100% den Stillstand des Wachstums und 0% ein Wachstum entsprechend dem der unbehandelten Kontrollpflanzen.

*Tabelle 6:*

*Wuchshemmung an Rasen*

| Verbindung gem. Beispiel | Anwendungs-konz. (kg/ha) | Wuchs-hemmung (%) | Phytotox. Wirkung |
|---|---|---|---|
| 3 | 0,62<br>0,31 | 82<br>70 | keine Schäden |
| Vergleich: Maleinsäurehydrazid | 2,50 | 60 | starke Schäden |

6. *Erhöhung des Zuckergehaltes bei Zuckerrohr*

*Untersuchungsverfahren*

Zuckerrohrpflanzen werden unter Gewächshausbedingungen bei 25 bis 35° C und ca. 65% Luftfeuchtigkeit angezogen. Unterschiedliche Mengen der formulierten Mittel wurden in Wasser suspendiert, das zusätzlich etwa 0,25 Gew.-% eines oberflächenaktiven Mittels (Nonylphenol) enthielt.

Jeweils 0,3 ml der Suspension wurden mit Hilfe einer Spritze in den Spindelbereich in Höhe der

letzten sichtbaren Blattspreite *(dewlap)* appliziert (10 Pflanzen pro Konzentration). Von den behandelten Pflanzen sowie von den nicht behandelten Kontrollen wurden nach 3 Wochen bei der Ernte die Blätter entfernt und die Internodien gruppenweise auf ihren Saccharosegehalt analysiert. Die Ergebnisse sind in Tabelle 7 dargestellt.

*Tabelle 7*

| Verbindung gem. Beispiel | Anwendungs-konz. (kg/ha) | Zuckergehalt (%) bei der Ernte |
|---|---|---|
| 3 | 0,62 | 145 |
| 19 | 0,62 | 239 |
| Kontrolle | | 100 |

7. *Anregung der Ethylenfreisetzung*

Calamondinorangen wurden 2 Minuten lang in Wirkstofflösung von 2000 ppm Wirkstoffgehalt getaucht. Das von den Früchten gebildete Ethylen wurde danach täglich 5 Tage lang gaschromatisch erfasst.

Die Ergebnisse der Tabelle 8 stellen einen Mittelwert aus insgesamt 3 Versuchsreihen dar.

*Tabelle 8*

| Verbindung gem. Beispiel | Ethylenproduktion (rel. Einh.) 1 bis 5 d gesamt |
|---|---|
| Kontrolle | 2,4 |
| 3 | 12,4 |
| 10 | 9,0 |
| Vergleich Glyoxim | 3,8 |

Die anspruchsgemässen Verbindungen wiesen eine wesentlich höhere Initial- und Dauerwirkung bei der Ethylenfreisetzung als das Vergleichsmittel auf. Auch die insgesamt gebildete Ethylenmenge übersteigt deutlich die des Vergleichsmittels. Da Ethylen, das auch von der Pflanze gebildet wird, massgeblich am Reifungs- und Abszissionsprozess beteiligt ist, dient der Versuch als Beweis für die durch die anmeldungsgemässen Verbindungen hervorgerufene beschleunigte und umfassende Ausbildung von Trennungsgewebe und damit für die Einleitung des Abszissionsvorgangs.

**Patentansprüche** für die Vertragsstaaten BE, CH, DE, FR, GB, IT, LI, LU, NL, SE

1. Verbindungen der Formel I

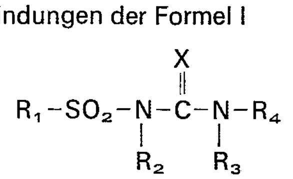

(I)

worin

$R_1$ einen gesättigten cycloaliphatischen Rest mit 3 bis 12 C-Atomen oder einen ein- oder mehrfach ungesättigten cycloaliphatischen Rest mit 5 bis 12 C-Atomen, die ggf. durch bis zu 4 Halogenatome und/oder durch einen oder mehrere $(C_1-C_4)$-Alkyl- oder Halogenalkyl (letztere mit 1 bis 3 Halogenatomen) bzw. durch einen $(C_1-C_4)$-Alkoxycarbonylrest substituiert sein können; einen bicyclischen gesättigten oder 1- oder 2fach ungesättigten aliphatischen Rest mit 7 bis 12 C-Atomen, welcher ggf. bis zu 6 Halogenatome oder eine oder mehrere $(C_1-C_4)$-Alkylreste tragen kann oder in dem eine $-CH_2-$ Brücke durch Sauerstoff ersetzt sein kann,

$R_2$, $R_3$, H oder $(C_1-C_4)$-Alkyl,

X O oder S, und

$R_4$ einen 2 bis 3 Stickstoffatome enthaltenden heterocyclischen Sechsring, der ggf. 1- bis 3fach durch Halogen, $NO_2$, CN, CHO, $(C_1-C_4)$-Alkylamino, $(C_1-C_4)$-Dialkylamino, einen (ggf. durch Halogen, $(C_1-C_3)$-Alkoxy, $(C_1-C_3)$-Alkylthio, $(C_1-C_3)$-Alkylamino, $(C_1-C_3)$-Dialkylamino oder $(C_1-C_4)$-Alkoxycarbonyl substituierten) $(C_1-C_4)$-Alkylrest, einen (ggf. durch Halogen oder $(C_1-C_4)$-Alkoxycarbonyl substituierten) $(C_1-C_4)$-Alkoxy- oder $(C_1-C_4)$-Alkylthiorest oder $(C_1-C_4)$-Alkoxycarbonyl substituiert ist, bedeuten,

sowie, falls $R_2$ Wasserstoff bedeutet, deren physiologisch verträgliche Salze mit Basen.

2. Verbindungen gemäss Anspruch 1, worin $R_2$ und $R_3$ Wasserstoff und X Sauerstoff bedeuten.

3. Verbindungen gemäss einem der Ansprüche 1 oder 2, worin $R_1$ einen durch Chlor oder Brom substituierten Cycloalkylrest mit 6 bis 12 C-Atomen bedeutet.

4. Verbindungen gemäss einem der Ansprüche 1 oder 2, worin $R_1$ einen Cyclohexenyl, Methylcyclohexenyl oder Chlorcyclohexenylrest bedeutet.

5. Verbindungen gemäss einem der Ansprüche 1 oder 2, worin $R_1$ einen Cycloheptenyl oder Oxabicyclo-[2.2.1]-heptenylrest bedeutet.

6. Verbindungen gemäss einem der Ansprüche 1 oder 2, worin $R_1$ einen Chlorbicyclo-[2.2.1]-heptylrest bedeutet.

7. Verbindungen gemäss einem der Ansprüche 1 bis 6, worin $R_4$ einen in 4- und 6-Stellung durch Methyl und/oder Methoxy substituierten Pyrimidin- oder s-Triazinring bedeutet.

8. Verbindung der Formel

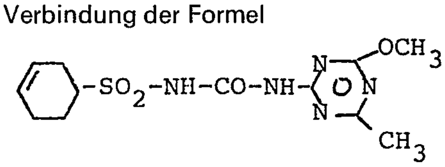

9. Verbindung der Formel

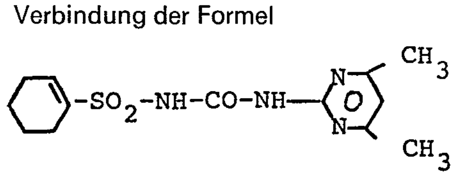

10. Verbindung der Formel

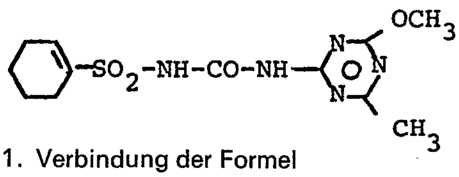

11. Verbindung der Formel

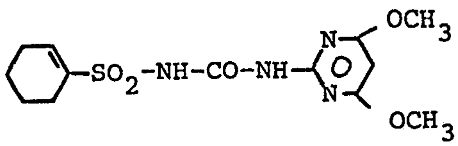

12. Verbindung der Formel

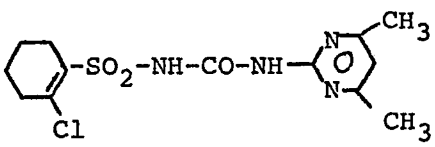

13. Verbindung der Formel

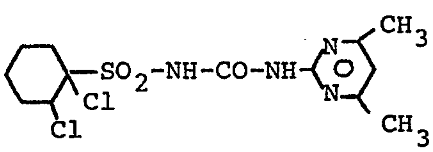

14. Verbindung der Formel

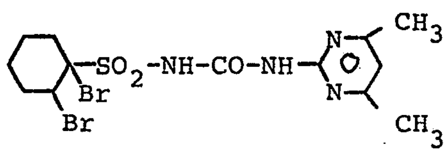

15. Verfahren zur Herstellung von Verbindungen der Formel I, dadurch gekennzeichnet, dass man

a) Verbindungen der Formel

$$R_1-SO_2-N=C=O \qquad (II)$$

oder

$$R_1-SO_2-N(R_2)-C(=X)-Cl \qquad (III)$$

mit Verbindungen der Formel

$$HN(R_3)-R_4 \qquad (IV)$$

oder

b) Verbindungen der Formel

$$R_1-SO_2-NH(R_2) \qquad (V)$$

mit Verbindungen der Formel

$$S=C=N-R_4 \qquad (VI)$$

oder

$$Cl-C(=X)-N(R_3)-R_4 \qquad (VII)$$

worin $R_3$ $(C_1$-$C_4)$-Alkyl bedeutet, oder

$$R_5-C(R_6)=C(R_7)-SO_2N(R_2)-C(=X)-N(R_3)-R_4 \qquad (VIII)$$

worin $R_5$, $R_6$ und $R_7$ Wasserstoff, Halogen oder $(C_1$-$C_4)$-Alkyl bedeuten mit Verbindungen der Formel

$$R_8-C(R_9)=C(R_{10})-C(R_{11})=C(R_{12})-R_{13} \qquad (IX)$$

worin $R_8$ bis $R_{13}$ für Wasserstoff, Halogen oder $CH_3$ stehen und einer der Reste $R_9$ bis $R_{12}$ auch $(C_2$-$C_4)$-Alkyl oder $(C_1$-$C_4)$-Alkoxycarbonyl sein kann oder $R_8$ und $R_{13}$ zusammen eine $(C_1$-$C_6)$-Alkylengruppe, in der eine $-CH_2$-Gruppe auch durch Sauerstoff ersetzt sein kann, darstellen, umsetzt.

16. Herbizide Mittel, gekennzeichnet durch einen Gehalt an Verbindungen der Formel I.

17. Wachstumsregulierende Mittel, gekennzeichnet durch einen wirksamen Gehalt an einer Verbindung der Formel I.

18. Herbizide Mittel, gekennzeichnet durch einen wirksamen Gehalt an einer Verbindung gemäss einem der Ansprüche 2 bis 14.

19. Wachstumsregulierende Mittel, gekennzeichnet durch einen wirksamen Gehalt an einer Verbindung gemäss einem der Ansprüche 2 bis 14.

20. Verwendung von Verbindungen der Formel I zur Bekämpfung von unerwünschtem Pflanzenwuchs.

21. Verwendung von Verbindungen gemäss einem der Ansprüche 2 bis 14 zur Bekämpfung von unerwünschtem Pflanzenwuchs.

22. Verwendung von Verbindungen der Formel I zur Wachstumsregulierung von Nutzpflanzen.

23. Verwendung von Verbindungen gemäss einem der Ansprüche 2 bis 14 zur Wachstumsregulierung von Nutzpflanzen.

24. Verfahren zur Bekämpfung von unerwünschtem Pflanzenwuchs, dadurch gekennzeichnet, dass man auf die zu bekämpfenden Pflanzen oder die Anbaufläche eine wirksame Menge einer Verbindung gemäss Formel I aufbringt.

25. Verfahren zur Wachstumsregulierung von Nutzpflanzen, dadurch gekennzeichnet, dass man auf die zu behandelnden Pflanzen eine wirksame Menge einer Verbindung gemäss Formel I aufbringt.

26. Verfahren zur Bekämpfung von unerwünschtem Pflanzenwuchs, dadurch gekennzeichnet, dass man die zu bekämpfenden Pflanzen oder die Anbaufläche eine wirksame Menge einer Verbindung gemäss einem der Ansprüche 2 bis 14 aufbringt.

27. Verfahren zur Wachstumsregulierung von Nutzpflanzen, dadurch gekennzeichnet, dass man auf die zu bekämpfenden Pflanzen oder die Anbaufläche eine wirksame Menge einer Verbindung gemäss einem der Ansprüche 2 bis 14 aufbringt.

**Patentansprüche** für den Vertragsstaat: AT

1. Herbizide bzw. wachstumsregulierende Mittel, gekennzeichnet durch einen Gehalt an einer Verbindung der Formel I

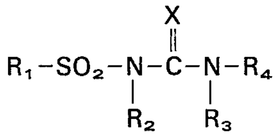 (I)

worin

$R_1$ einen gesättigten cycloaliphatischen Rest mit 3 bis 12 C-Atomen oder einen ein- oder mehrfach ungesättigten cycloaliphatischen Rest mit 5 bis 12 C-Atomen, die ggf. durch bis zu 4 Halogenatome und/oder durch einen oder mehrere $(C_1-C_4)$-Alkyl- oder Halogenalkyl (letztere mit 1 bis 3 Halogenatomen) bzw. durch einen $(C_1-C_4)$-Alkoxycarbonylrest substituiert sein können; einen bicyclischen gesättigten oder 1- oder 2fach ungesättigten aliphatischen Rest mit 7 bis 12 C-Atomen, welcher ggf. bis zu 6 Halogenatome oder eine oder mehrere $(C_1-C_4)$-Alkylreste tragen kann oder in dem eine $CH_2$-Brücke durch Sauerstoff ersetzt sein kann,

$R_2$, $R_3$, H oder $(C_1-C_4)$-Alkyl,

X O oder S, und

$R_4$ einen 2 bis 3 Stickstoffatome enthaltenden heterocyclischen Sechsring, der ggf. 1- bis 3fach durch Halogen, $NO_2$, CN, CHO, $(C_1-C_4)$-Alkylamino, $(C_1-C_4)$-Dialkylamino, einen (ggf. durch Halogen, $(C_1-C_3)$-Alkoxy, $(C_1-C_3)$-Alkylthio, $(C_1-C_3)$-Alkylamino, $(C_1-C_3)$-Dialkylamino oder $(C_1-C_4)$-Alkoxycarbonyl substituierten) $(C_1-C_4)$-Alkylrest, einen (ggf. durch Halogen oder $(C_1-C_4)$-Alkoxycarbonyl substituierten) $(C_1-C_4)$-Alkoxy- oder $(C_1-C_4)$-Alkylthiorest oder $(C_1-C_4)$-Alkoxycarbonyl substituiert ist, bedeuten,

sowie, falls $R_2$ Wasserstoff bedeutet, deren physiologisch verträgliche Salze mit Basen.

2. Verwendung von Verbindungen der Formel I zur Bekämpfung von Schadpflanzen bzw. zur Wachstumsregulierung von Nutzpflanzen.

**Claims** for the Contracting States BE, CH, DE, FR, GB, IT, LI, LU, NL, SE

1. A compound of the Formula (I):

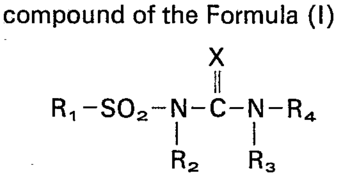 (I)

wherein

$R_1$ denotes a saturated cycloaliphatic radical having 3 to 12 C-atoms or a cycloaliphatic radical having 5 to 12 C-atoms, which is mono- or polyunsaturated and which C-atoms can optionally be substituted by up to 4 halogen atoms and/or by one or more $(C_1-C_4)$-alkyl or -halogenoalkyl (the latter having 1 to 3 halogen atoms) or by a $(C_1-C_4)$-alkoxycarbonyl radical; a bicyclic saturated or mono- or polyunsaturated aliphatic radical having 7 to 12 C-atoms, which can optionally carry up to 6 halogen atoms or one or more $(C_1-C_4)$-alkyl radicals or in which a $-CH_2-$ bridge can be replaced by oxygen,

$R_2$ and $R_3$ denote H or $(C_1-C_4)$-alkyl,

X denotes O or S,

$R_4$ denotes a six-membered heterocyclic ring containing 2 or 3 nitrogen atoms, which is optionally substituted 1 to 3 times by halogen, $NO_2$, CN, CHO, $(C_1-C_4)$-alkylamino, $(C_1-C_4)$-dialkylamino, a $(C_1-C_4)$-alkyl radical [which is optionally substituted by halogen, $(C_1-C_3)$-alkoxy, $(C_1-C_3)$-alkylthio, $(C_1-C_3)$-alkylamino, $(C_1-C_3)$-dialkylamino or $(C_1-C_4)$-alkoxycarbonyl], a $(C_1-C_4)$-alkoxy or $(C_1-C_4)$-alkylthio radical [which is optionally substituted by halogen or $(C_1-C_4)$-alkoxycarbonyl], or $(C_1-C_4)$-alkoxycarbonyl,

and, if $R_2$ denotes hydrogen, its physiologically tolerated salts with bases.

2. A compound as claimed in Claim 1, wherein $R_2$ and $R_3$ denote hydrogen and X denotes oxygen.

3. A compound as claimed in one of Claims 1 or 2, wherein $R_1$ denotes a cycloalkyl radical having 6 to 12 C-atoms which is substituted by chlorine or bromine.

4. A compound as claimed in one of Claims 1 or 2, wherein $R_1$ denotes a cyclohexenyl, methylcyclohexenyl or chlorocyclohexenyl radical.

5. A compound as claimed in one of Claims 1 or 2, wherein $R_1$ denotes a cycloheptenyl or oxabicyclo-[2.2.1]-heptenyl radical.

6. A compound as claimed in one of Claims 1 or 2, wherein $R_1$ denotes a chlorobicyclo-[2.2.1]-heptyl radical.

7. A compound as claimed in one of Claims 1 to 6, wherein $R_4$ denotes a pyrimidine or s-triazine ring which is substituted in the 4- and 6-position by methyl and/or methoxy.

8. The compound of the formula

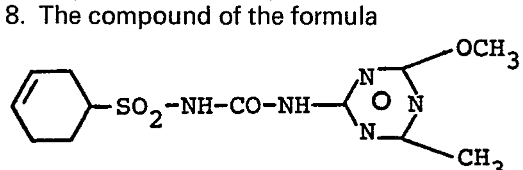

9. The compound of the formula

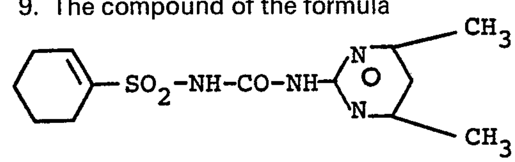

10. The compound of the formula

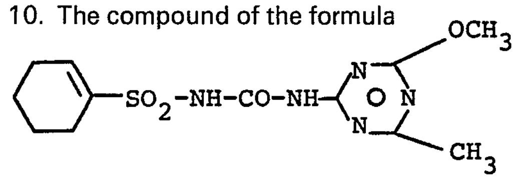

11. The compound of the formula

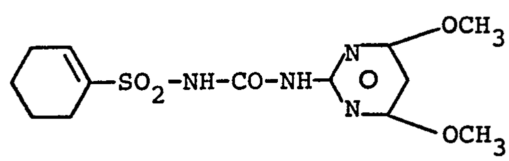

12. The compound of the formula

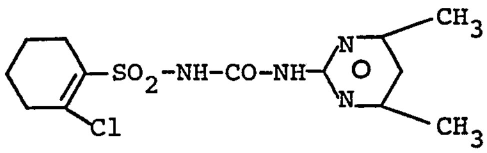

13. The compound of the formula

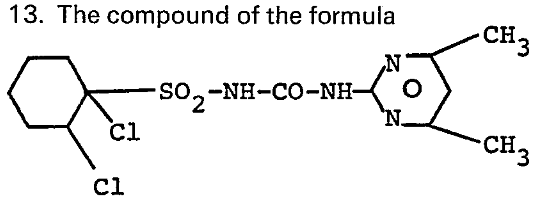

14. The compound of the formula

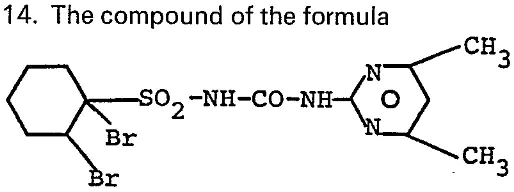

15. A process for the preparation of compounds of the Formula (I) which comprises
(*a*) reacting compounds of the formula

$$R_1-SO_2-N=C=O \qquad (II)$$

or

$$R_1-SO_2-\underset{\underset{R_2}{|}}{N}-\overset{\overset{X}{\|}}{C}-Cl \qquad (III)$$

with compounds of the formula

$$H\underset{\underset{R_3}{|}}{N}-R_4 \qquad (IV)$$

or
(*b*) reacting compounds of the formula

$$R_1-SO_2-\underset{\underset{R_2}{|}}{N}H \qquad (V)$$

with compounds of the formula

$$S=C=N-R_4 \qquad (VI)$$

or

$$Cl-\overset{\overset{X}{\|}}{C}-\underset{\underset{R_3}{|}}{N}-R_4 \qquad (VII)$$

wherein $R_3$ denotes $(C_1\text{-}C_4)$-alkyl
or
(*c*) reacting compounds of the formula

$$R_5-\overset{\overset{R_6}{|}}{C}=\overset{\overset{R_7}{|}}{C}-SO_2\underset{\underset{R_2}{|}}{N}-\overset{\overset{X}{\|}}{C}-\underset{\underset{R_3}{|}}{N}-R_4 \qquad (VIII)$$

wherein $R_5$, $R_6$ and $R_7$ denote hydrogen, halogen or $(C_1\text{-}C_4)$-alkyl
with compounds of the formula

$$R_8-\overset{\overset{R_9}{|}}{C}=\overset{\overset{R_{10}}{|}}{C}-\overset{\overset{R_{11}}{|}}{C}=\overset{\overset{R_{12}}{|}}{C}-R_{13} \qquad (IX)$$

wherein $R_8$ to $R_{13}$ represent hydrogen, halogen or $CH_3$ and one of the radicals $R_9$ to $R_{12}$ can also be $(C_2\text{-}C_4)$-alkyl or $(C_1\text{-}C_4)$-alkoxycarbonyl or $R_8$ and $R_{13}$ together represent a $(C_1\text{-}C_6)$-alkylene group, in which a $-CH_2-$ group can also be replaced by oxygen.

16. A herbicidal agent containing compounds of the Formula (I).

17. A plant-growth regulating agent having an effective content of a compound of the Formula (I).

18. A herbicidal agent having an effective content of a compound as claimed in one of Claims 2 to 14.

19. A plant-growth regulating agent having an effective content of a compound as claimed in one of Claims 2 to 14.

20. The use of compounds of the Formula (I) for controlling undesired plant growth.

21. The use of compounds as claimed in one of Claims 2 to 14 for controlling undesired plant growth.

22. The use of compounds of the Formula (I) for regulating the growth of crop plants.

23. The use of compounds as claimed in one of Claims 2 to 14 for regulating the growth of crop plants.

24. A process for controlling undesired plant growth which comprises applying an effective amount of a compound according to Formula (I) to the plants to be controlled or to the area of cultivation.

25. A process for regulating the growth of crop plants which comprises applying an effective amount of a compound according to Formula (I) to the plants to be treated.

26. A process for controlling undesired plant growth which comprises applying an effective amount of a compound as claimed in one of Claims 2 to 14 to the plants to be controlled or to the area of cultivation.

27. A process for regulating the growth of crop plants which comprises applying an effective amount of a compound as claimed in one of Claims 2 to 14 to the plants to be controlled or to the area of cultivation.

**Claims** for the Contracting State: AT

1. Herbicidal and plant-growth regulating agents, respectively comprising a compound of Formula (I)

$$R_1-SO_2-\underset{\underset{R_2}{|}}{N}-\overset{\overset{X}{\|}}{C}-\underset{\underset{R_3}{|}}{N}-R_4 \qquad (I)$$

wherein

$R_1$ denotes a saturated cycloaliphatic radical having 3 to 12 C-atoms or a cycloaliphatic radical

having 5 to 12 C-atoms, which is mono- or polyunsaturated and which C-atoms can optionally be substituted by up to 4 halogen atoms and/or by one or more ($C_1$-$C_4$)-alkyl or -halogenoalkyl (the latter having 1 to 3 halogen atoms) or by a ($C_1$-$C_4$)-alkoxycarbonyl radical; a bicyclic saturated or mono- or polyunsaturated aliphatic radical having 7 to 12 C-atoms, which can optionally carry upt to 6 halogen atoms or one or more ($C_1$-$C_4$)-alkyl radicals or in which a $-CH_2-$ bridge can be replaced by oxygen,

$R_2$ and $R_3$ denote H or ($C_1$-$C_4$)-alkyl,

X denotes O or S,

$R_4$ denotes a six-membered heterocyclic ring containing 2 or 3 nitrogen atoms, which is optionally substituted 1 to 3 times by halogen, $NO_2$, CN, CHO, ($C_1$-$C_4$)-alkylamino, ($C_1$-$C_4$)-dialkylamino, a ($C_1$-$C_4$)-alkyl radical [which is optionally substituted by halogen, ($C_1$-$C_3$)-alkoxy, ($C_1$-$C_3$)-alkylthio, ($C_1$-$C_3$)-alkylamino, ($C_1$-$C_3$)-dialkylamino or ($C_1$-$C_4$)-alkoxycarbonyl], a ($C_1$-$C_4$)-alkoxy or ($C_1$-$C_4$)-alkylthio radical [which is optionally substituted by halogen or ($C_1$-$C_4$)-alkoxycarbonyl], or ($C_1$-$C_4$)-alkoxycarbonyl,

and, if $R_2$ denotes hydrogen, its physiologically tolerated salts with bases.

2. The use of compounds of the Formula (I) for controlling undesired plant growth and for regulating the growth of crop plants.

**Revendications** pour les Etats contractants BE, CH, DE, FR, GB, IT, LI, LU, NL, SE

1. Composés répondant à la formule (I)

$$R_1-SO_2-\underset{R_2}{\underset{|}{N}}-\overset{X}{\overset{\|}{C}}-\underset{R_3}{\underset{|}{N}}-R_4 \qquad (I)$$

dans laquelle

$R_1$ représente un radical cycloaliphatique saturé contenant de 3 à 12 atomes de carbone ou un radical cycloaliphatique à une ou plusieurs insaturations qui contient de 5 à 12 atomes de carbone, chacun de ces radicaux pouvant éventuellement porter au plus 4 atomes d'halogène et/ou un ou plusieurs alkyles en $C_1$-$C_4$ ou halogénoalkyles (ces derniers avec de 1 à 3 atomes d'halogène) ou un alcoxycarbonyle dont l'alcoxy contient de 1 à 4 atomes de carbone; un radical aliphatique bicyclique en $C_7$-$C_{12}$, saturé ou à une ou deux insaturations, qui peut porter au plus 6 atomes d'halogène ou un ou plusieurs alkyles en $C_1$-$C_4$ ou dans lequel un pont $-CH_2-$ peut être remplacé par un atome d'oxygène,

$R_2$ et $R_3$ représentent chacun H ou un alkyle en $C_1$-$C_4$,

X représente O ou S, et

$R_4$ représente un noyau hexagonal hétérocyclique contenant 2 ou 3 atomes d'azote, noyau qui porte éventuellement de un à trois substituants pris dans l'ensemble constitué par les halogènes et les radicaux $NO_2$, CN, CHO, alkylamino en $C_1$-$C_4$ et di-($C_1$-$C_4$)-alkylamino, un radical alkyle en $C_1$-$C_4$ (éventuellement porteur d'un halogène, d'un alcoxy en $C_1$-$C_3$, d'un alkylthio en $C_1$-$C_3$, d'un alkylamino en $C_1$-$C_3$, d'un di-($C_1$-$C_3$)-alkylamino ou d'un ($C_1$-$C_4$)-alcoxycarbonyle), un radical alcoxy en $C_1$-$C_4$ ou alkylthio en $C_1$-$C_4$ (éventuellement porteur d'un halogène ou d'un alcoxycarbonyle dont l'alcoxy contient de 1 à 4 atomes de carbone) ou un radical alcoxycarbonyle dont la partie alcoxy contient de 1 à 4 atomes de carbone,

et aussi, lorsque $R_2$ représente l'hydrogène, les sels acceptables du point de vue physiologique que ces composés forment avec des bases.

2. Composés selon la revendication 1, dans lesquels $R_2$ et $R_3$ représentent chacun l'hydrogène et X représente l'oxygène.

3. Composés selon l'une des revendications 1 ou 2, dans lesquels $R_1$ représente un radical cycloalkyle porteur d'un atome de chlore ou de brome et contenant de 6 à 12 atomes de carbone.

4. Composés selon l'une des revendications 1 ou 2, dans lesquels $R_1$ représente un radical cyclohexényle, méthylcyclohexényle ou chlorocyclohexényle.

5. Composés selon l'une des revendications 1 ou 2, dans lesquels $R_1$ représente un radical cycloheptényle ou un radical oxabicyclo-[2.2.1]-hepténNyle.

6. Composés selon l'une des revendications 1 ou 2, dans lesquels $R_1$ représente un radical chlorobicyclo-[2.2.1]-heptyle.

7. Composés selon l'une des revendications 1 à 6, dans lesquels $R_4$ représente un radical pyrimidyle ou s-triazinyle portant un radical méthyle et/ou un radical méthoxy aux positions 4 et 6.

8. Composé de formule

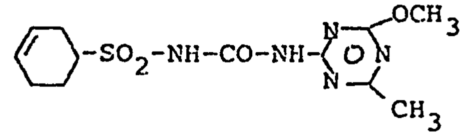

9. Composé de formule

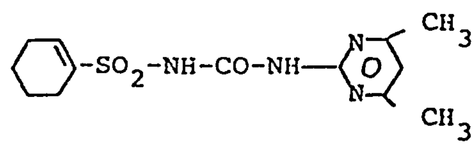

10. Composé de formule

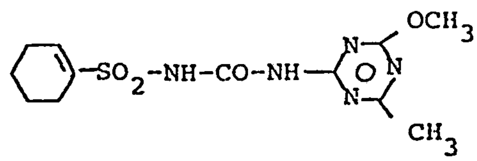

11. Composé de formule

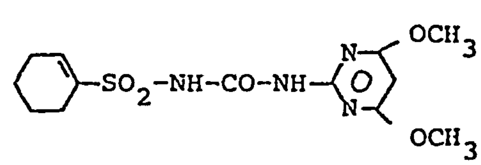

12. Composé de formule

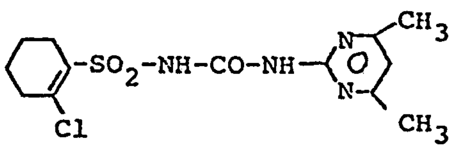

13. Composé de formule

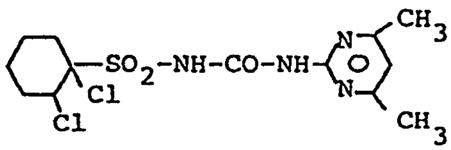

14. Composé de formule

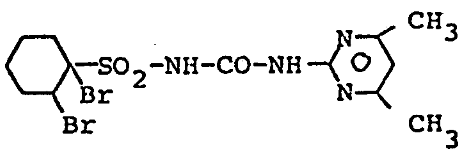

15. Procédé pour préparer des composés de formule (I), caractérisé en ce que
a) on fait réagir des composés de formule

$$R_1-SO_2-N=C=O \qquad (II)$$

ou de formule

$$R_1-SO_2-\underset{R_2}{\underset{|}{N}}-\overset{X}{\overset{\|}{C}}-Cl \qquad (III)$$

avec des composés de formule

$$H\underset{R_3}{\underset{|}{N}}-R_4 \qquad (IV)$$

ou
b) on fait réagir des composés de formule

$$R_1-SO_2-\underset{R_2}{\underset{|}{NH}} \qquad (V)$$

avec des composés de formule

$$S=C=N-R_4 \qquad (VI)$$

ou de formule

$$Cl-\overset{X}{\overset{\|}{C}}-\underset{R_3}{\underset{|}{N}}-R_4 \qquad (VII)$$

dans laquelle $R_3$ représente un radical alkyle contenant de 1 à 4 atomes de carbone,
ou
c) on fait réagir des composés répondant à la formule

$$R_5-\overset{R_6}{\overset{|}{C}}=\overset{R_7}{\overset{|}{C}}-SO_2\underset{R_2}{\underset{|}{N}}-\overset{X}{\overset{\|}{C}}-\underset{R_3}{\underset{|}{N}}-R_4 \qquad (VIII)$$

dans laquelle $R_5$, $R_6$ et $R_7$ représentent chacun un atome d'hydrogène ou d'halogène ou un radical alkyle en $C_1$-$C_4$, avec des composés répondant à la formule

$$R_8-\overset{R_9}{\overset{|}{C}}=\overset{R_{10}}{\overset{|}{C}}-\overset{R_{11}}{\overset{|}{C}}=\overset{R_{12}}{\overset{|}{C}}-R_{13} \qquad (IX)$$

dans laquelle $R_8$ à $R_{13}$ représentent chacun l'hydrogène, un halogène ou un radical méthyle et l'un des symboles $R_9$ à $R_{12}$ peut également représenter un alkyle en $C_2$-$C_4$ ou un $(C_1$-$C_4)$-alcoxycarbonyle, ou $R_8$ et $R_{13}$ représentent ensemble un radical alkylène en $C_1$-$C_6$ dans lequel un radical $-CH_2-$ peut être remplacé par un atome d'oxygène.

16. Produits herbicides, caractérisés en ce qu'ils contiennent des composés de formule (I).

17. Produits régulateurs de croissance, caractérisés en ce qu'ils contiennent une quantité efficace d'un composé de formule (I).

18. Produits herbicides, caractérisés en ce qu'ils contiennent une quantité efficace d'un composé selon l'une des revendications 2 à 14.

19. Produits régulateurs de croissance, caractérisés en ce qu'ils contiennent une quantité efficace d'un composé selon l'une des revendications 2 à 14.

20. Application de composés de formule (I) pour combattre la végétation indésirable.

21. Application de composés selon l'une des revendications 2 à 14 pour combattre la végétation indésirable.

22. Application de composés de formule (I) pour régler la croissance de plantes utiles.

23. Application de composés selon l'une des revendications 2 à 14 pour régler la croissance de plantes utiles.

24. Procédé pour combattre la végétation indésirable, caractérisé en ce qu'on applique une quantité efficace d'un composé de formule (I) sur les plantes à combattre ou sur la surface cultivée.

25. Procédé pour régler la croissance de plantes utiles, caractérisé en ce qu'on applique une quantité efficace d'un composé de formule (I) sur les plantes à traiter.

26. Procédé pour combattre la végétation indésirable, caractérisé en ce qu'on applique une quantité efficace d'un composé selon l'une des revendications 2 à 14 sur les plantes à combattre ou sur la surface cultivée.

27. Procédé pour régler la croissance de plantes utiles, caractérisé en ce qu'on applique une quantité efficace d'un composé selon l'une des revendications 2 à 14 sur les plantes à combattre ou sur la surface cultivée.

**Revendications** pour l'Etat contractant: AT

1. Produits herbicides et régulateurs de croissance, caractérisés en ce qu'ils contiennent un composé répondant à la formule (I)

$$R_1-SO_2-\underset{R_2}{\underset{|}{N}}-\overset{X}{\overset{\|}{C}}-\underset{R_3}{\underset{|}{N}}-R_4 \qquad (I)$$

dans laquelle

$R_1$ représente un radical cycloaliphatique saturé contenant de 3 à 12 atomes de carbone ou un radical cycloaliphatique à une ou plusieurs insaturations qui contient de 5 à 12 atomes de carbone, chacun de ces radicaux pouvant éventuellement porter au plus 4 atomes d'halogène et/ou un ou plusieurs alkyles en $C_1$-$C_4$ ou halogénoalkyles (ces derniers avec de 1 à 3 atomes d'halogène) ou un alcoxycarbonyle dont l'alcoxy contient de 1 à 4 atomes de carbone; un radical aliphatique bicyclique en $C_7$-$C_{12}$, saturé ou à une ou deux insaturations, qui peut porter au plus 6 atomes d'halogène ou un ou plusieurs alkyles en $C_1$-$C_4$ ou dans lequel un pont $-CH_2-$ peut être remplacé par un atome d'oxygène,

$R_2$ et $R_3$ représentent chacun H ou un alkyle en $C_1$-$C_4$,

X représente O ou S, et

$R_4$ représente un noyau hexagonal hétérocyclique contenant 2 ou 3 atomes d'azote, noyau qui porte éventuellement de un à trois substituants pris dans l'ensemble constitué par les halogènes et les radicaux $NO_2$, CN, CHO, alkylamino en $C_1$-$C_4$ et di-($C_1$-$C_4$)-alkylamino, un radical alkyle en $C_1$-$C_4$ (éventuellement porteur d'un halogène, d'un alcoxy en $C_1$-$C_3$, d'un alkylthio en $C_1$-$C_3$, d'un alkylamino en $C_1$-$C_3$, d'un di-($C_1$-$C_3$)-alkylamino ou d'un ($C_1$-$C_4$)-alcoxycarbonyle), un radical alcoxy en $C_1$-$C_4$ ou alkylthio en $C_1$-$C_4$ (éventuellement porteur d'un halogène ou d'un alcoxycarbonyle dont l'alcoxy contient de 1 à 4 atomes de carbone) ou un radical alcoxycarbonyle dont la partie alcoxy contient de 1 à 4 atomes de carbone,

ou encore, lorsque $R_2$ représente l'hydrogène, un sel acceptable du point de vue physiologique qu'un tel composé forme avec une base.

2. Application de composés de formule (I) pour combattre des plantes nuisibles ou pour régler la croissance de plantes utiles.